(19) Europäisches Patentamt
European Patent Office
Office européen des brevets

(11) Veröffentlichungsnummer: **0 027 199**
**B2**

# (12) NEUE EUROPÄISCHE PATENTSCHRIFT

(45) Veröffentlichungstag der neuen Patentschrift: **19.06.85**

(51) Int. Cl.⁴: **C 07 F 9/38,** A 61 K 31/66

(21) Anmeldenummer: **80105876.9**

(22) Anmeldetag: **27.09.80**

(54) **Verfahren zur Herstellung von Phosphono-hydroxy-essigsäure und Salzen derselben sowie diese enthaltende autivirale Mittel.**

(30) Priorität: **12.10.79 DE 2941384**

(43) Veröffentlichungstag der Anmeldung:
**22.04.81 Patentblatt 81/16**

(45) Bekanntmachung des Hinweises auf die Patenterteilung:
**25.05.83 Patentblatt 83/21**

(45) Bekanntmachung des Hinweises auf die Entscheidung über den Einspruch:
**19.06.85 Patentblatt 85/25**

(84) Benannte Vertragsstaaten:
**AT BE CH DE FR GB IT LI NL SE**

(56) Entgegenhaltungen:
**DE - A - 2 208 787**
**DE - A - 2 823 346**
**US - A - 3 032 500**
**US - A - 4 052 439**

**PHARMACOLOGY AND THERAPEUTICS, Band 4, 1979, Seiten 231-243 J.A. BOEZI: "The antiherpesvirus action of phosphonoacetate"**
**Leinbach et al., Biochemistry 15, pp. 426-430 (1976)**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Lieb, Folker, Dr., Alfred-Kubin-Strasse 1, D-5090 Leverkusen (DE)**
Erfinder: **Oediger, Hermann, Dr., Roggendorfstrasse 51, D-5000 Köln 80 (DE)**
Erfinder: **Streissle, Gert, Dr., Am Hochsitz 17, D-5600 Wuppertal (DE)**

**EP 0 027 199 B2**

BUNDESDRUCKEREI BERLIN

**Beschreibung**

Die vorliegende Erfindung betrifft Phosphono-hydroxy-essigsäure und/oder deren Salze enthaltende antivirale Mittel zur Verwendung in der Human- und Tiermedizin, sowie Verfahren zur Herstellung der Wirkstoffe.

Man kennt bereits antivirale Mittel, beispielsweise gegenüber Herpes simplex-Viren wirkende Mittel aus der Stoffklasse der Nucleoside, wie das 5-Jod-2'-desoxy-uridin (M. Negwer, Organisch-chemische Arzneimittel und ihre Synonyma, Seite 187, Nr. 1017; Akademie-Verlag, Berlin, 1978). Sie haben jedoch häufig unerwünschte Nebenwirkungen, wie mutagene, teratogene oder immunsuppressive Effekte.

Darüber hinaus ist beispielsweise aus der Reihe des Aminoadamantans das N-(1-Adamantyl)-2-(2-dimethyl-aminoethoxy)-acetamid bekanntgeworden (DE-OS 1 941 218). Es ist jedoch im Vergleich zu bekannten antiviralen Mitteln nur schwach wirksam.

Auch die Phosphonessigsäure ist als antivirales Mittel bekanntgeworden. Struktur-Wirkungsbeziehungen zeigen, daß die antivirale Wirkung der Phosphonessigsäure weitgehend an die unsubstituierte Methylengruppe geknüpft ist (Pharmacology and Therapeutics, Band 4, Seiten 231—243 [1979]).

Phosphono-hydroxy-essigsäure und seine Verwendung als Sequestrierungsmittel für Metallionen sind in der US-PS 3 032 500, insbesondere Beispiel 3, bereits beschrieben.

Es wurde nun gefunden, daß die Phosphono-hydroxy-essigsäure der Formel (I)

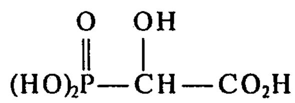

(I)

und/oder ihre physiologisch verträglichen Salze starke antivirale Eigenschaften aufweisen.

Weiterhin wurde gefunden, daß man die Phosphonohydroxyessigsäure der Formel (I) erhält, wenn man Verbindungen der Formel

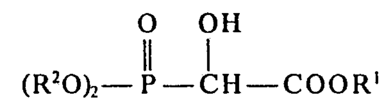

(IV)

in welcher
$R^1$ und $R^2$ gleich oder verschieden sein können und für Alkyl stehen oder gleich sind und für Aralkyl stehen,

a) im Falle, daß $R^1$ und $R^2$ für Alkyl stehen, entweder in Gegenwart einer Säure mit Wasser umsetzt oder nach Umsetzung mit Trialkylsilylhalogeniden bzw. mit Mono- oder Bistrialkylsilyl-niedrigalkylcarbonsäureamiden und darauf mit Trialkylsilylhalogeniden im wasserfreien Medium, anschließend mit Wasser in Gegenwart von Säuren bzw. von Basen umsetzt oder
b) im Falle, daß $R^1$ und $R^2$ für Aralkyl stehen, mit Wasserstoff in Gegenwart eines Edelmetallkatalysators umsetzt,

und gegebenenfalls die physiologisch verträglichen Salze herstellt.

Im einzelnen sind bei der Herstellung der Phosphonohydroxyessigsäure die folgenden Synthesestufen (inklusive Vorstufen) beteiligt.

Aldehyde der Formel (II)

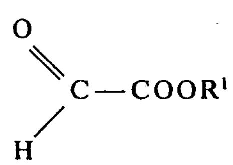

(II)

in welcher
$R^1$ für Alkyl oder Aralkyl steht,
werden mit einem Phosphit der Formel (III)

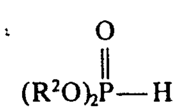

(III)

in welcher

$R^2$ für Alkyl oder Aralkyl steht,
umgesetzt und die erhaltene Verbindung der Formel (IV)

$$(R^2O)_2\overset{\overset{O}{\|}}{P}-\overset{\overset{OH}{|}}{CH}-COOR^1 \qquad (IV)$$

wird im Fall, daß $R^1$ und $R^2$ für Alkyl stehen,

a) mit Wasser in Anwesenheit einer Säure umgesetzt oder
b) mit einem Trialkylsilylhalogenid der Formel (V)

$$(R^3)_3Si-Hal$$

in der
$R^3$ für einen Alkylrest und
Hal für Chlor, Brom oder Jod steht,
umgesetzt, mit Wasser hydrolysiert, die erhaltene Verbindung der Formel (VI)

$$(HO)_2\overset{\overset{O}{\|}}{P}-\overset{\overset{OH}{|}}{CH}-CO_2R^1 \qquad (VI)$$

in der
$R^1$ die obige Bedeutung hat,
mit Wasser in Anwesenheit einer Säure oder Base umgesetzt oder

c) mit einem Mono- oder Bis-trialkylsilyl-niedrigalkylcarbonsäureamid als Silylierungsreagenz (VII, VIII) umgesetzt und die erhaltene Verbindung der Formel (IX)

$$(R^2O)_2\overset{\overset{O}{\|}}{P}-\overset{\overset{OSi(R^4)_3}{|}}{CH}-COOR^1 \qquad IX)$$

in welcher $R^4$ für Alkyl steht, mit einem Trialkylsilylhalogenid der Formel (V) umgesetzt, mit Wasser hydrolysiert und darauf mit Wasser in Anwesenheit einer Säure oder Base umgesetzt

oder für den Fall, daß
$R^1$ und $R^2$ in der Formel (IV) für Aralkyl stehen, mit Wasserstoff in Gegenwart eines Edelmetallkatalysators behandelt.

Überraschenderweise zeigt die Phosphono-hydroxy-essigsäure eine erheblich höhere und/oder breitere antivirale Wirksamkeit als die aus dem Stand der Technik bekannten antiviralen Wirkstoffe. Der erfindungsgemäße Stoff stellt somit eine Bereicherung der Pharmazie dar.

Nach dem Stand der Technik (Pharmacology and Therapeutics, Band 4, Seiten 231—243 [1979], Biochemistry 15, Seiten 426—430 [1976]) sollte eine an der Methylengruppe substituierte Phosphonessigsäure keine (bei Substitution durch Phenyl oder die $NH_2$-Gruppe) oder nur sehr geringe (bei Substitution durch die $CH_3$-Gruppe) antivirale Wirkung zeigen.

Verwendet man als Ausgangsstoffe Dimethylphosphit und Glyoxylsäurebutylester und zur Verseifung Trimethylsilyliodid, Wasser und Natronlauge, so kann der Reaktionsablauf durch das Reaktionsschema B wiedergegeben werden (s. S. 8, Reaktionsschema B).

Die als Ausgangsstoffe verwendeten Glyoxylsäureester sind bekannt (Organic Synthesis, Band 4, Seite 124) oder können nach bekannten Verfahren hergestellt werden.

In der Formel (II) steht vorzugsweise $R^1$ für Alkyl mit 1—8 Kohlenstoffatomen, insbesondere für Propyl und Butyl oder für Aralkyl, insbesondere für Benzyl.

Beispielsweise seien genannt:
Glyoxylsäurepropylester, Glyoxylsäurebutylester, Glyoxylsäurebenzylester.

Die als Ausgangsstoffe verwendeten Dialkyl- oder Diaralkylphosphite sind ebenfalls bekannt (Houben—Weyl, Methoden der organischen Chemie, Band XII/2, Seite 20).

In der Formel (III) steht vorzugsweise $R^2$ für Alkyl mit 1—4 Kohlenstoffatomen, insbesondere für Methyl und Ethyl und für Aralkyl, insbesondere für Benzyl.

Beispielsweise seien genannt:
Dimethylphosphit, Diethylphosphit, Dibenzylphosphit. Als Verdünnungsmittel kommen alle inerten organischen Lösungsmittel in Frage. Geeignete Lösungsmittel sind aromatische Kohlenwasserstoffe wie beispielsweise Toluol, acyclische oder cyclische Ether, beispielsweise Diethylether oder Dioxan, Alkohole, beispielsweise Methanol und Ethanol und aliphatische Carbonsäureamide, beispielsweise Dimethylformamid und aliphatische Carbonsäurenitrile, beispielsweise Acetonitril. Man kann jedoch auch ohne Verdünnungsmittel arbeiten.

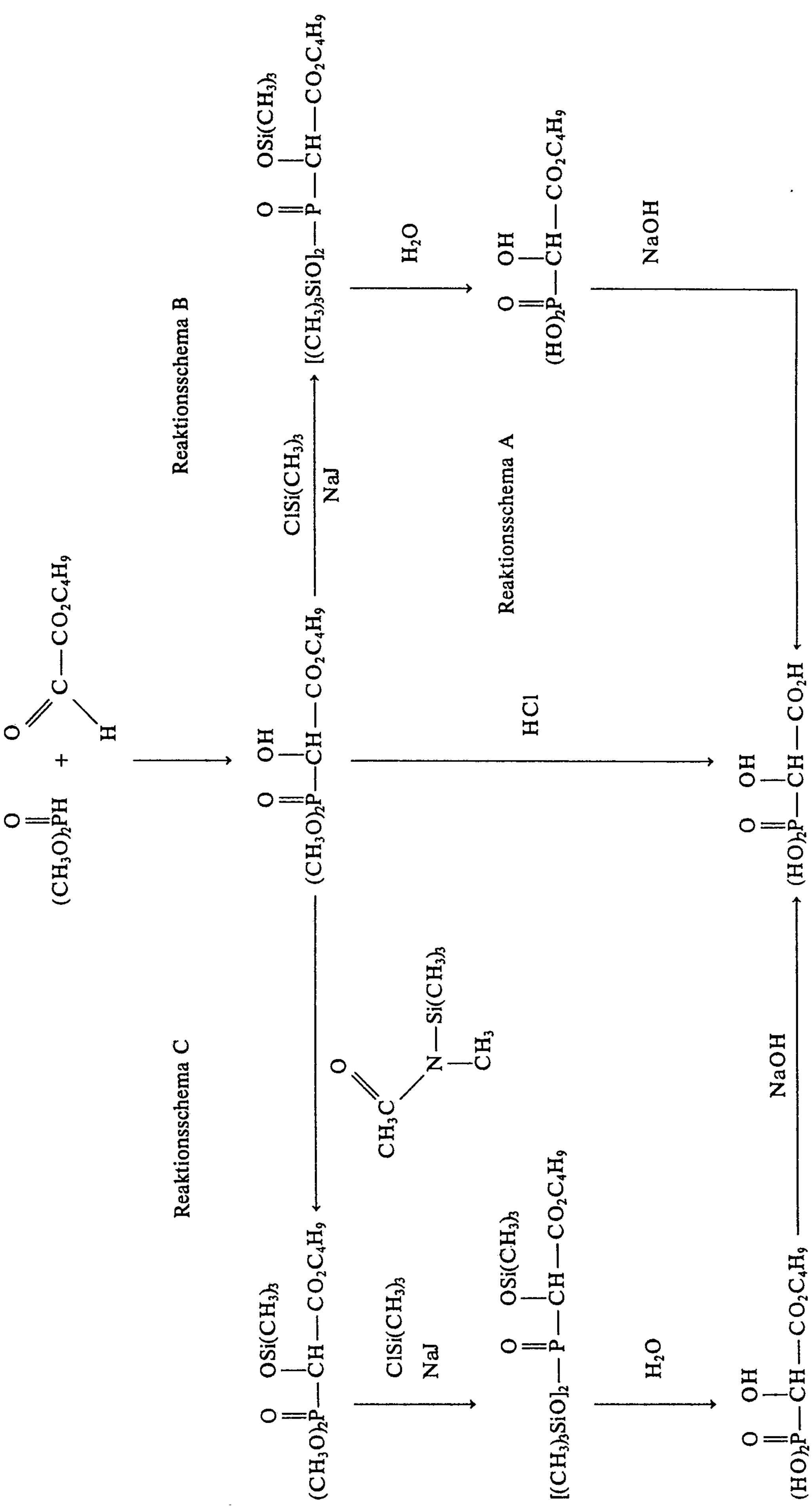
Reaktionsschema B
ClSi(CH$_3$)$_3$
NaJ
H$_2$O
NaOH
Reaktionsschema A
HCl
Reaktionsschema C
ClSi(CH$_3$)$_3$
NaJ
H$_2$O
NaOH

Die Umsetzung von Verbindungen der Formel (II) mit solchen der Formel (III) wird in Gegenwart einer Base vorgenommen. Vorzugsweise verwendet man Erdalkalihydroxide, Alkalihydroxide oder Alkalialkoholate, insbesondere Natriumhydroxid oder Natriummethylat.

Die Umsetzung von Verbindungen der Formel (II) mit solchen der Formel (III) wird bei Temperaturen zwischen 0 und 150° C, vorzugsweise zwischen 25 und 120° C durchgeführt. Die Reaktionsdauer ist von der Temperatur abhängig und liegt zwischen 1 und 30 Stunden.

Bei der Durchführung der Umsetzung von (II) mit (III) setzt man 1 Mol der Verbindung (II) mit 0,9 bis 1,1 Mol, vorzugsweise mit 0,95 bis 1,0 Mol der Verbindung (III) unter Zugabe von 0,01 bis 0,1 Mol Base zur Verbindung der Formel (IV) um.

Nach Stufe a) des Verfahrens werden (wie im Reaktionsschema A spezifiziert) die Verbindungen der allgemeinen Formel (IV), in der $R_1$ und $R_2$ Alkylreste darstellen, sauer verseift. Als Säuren verwendet man vorzugsweise wäßrige anorganische Säuren, beispielsweise Schwefelsäure oder Salzsäure, vorzugsweise Salzsäure.

Die Verseifung von (IV) zur Phosphonohydroxyessigsäure (I) wird in einem Temperaturbereich zwischen 20 und 120° C, vorzugsweise zwischen 90 und 110° C durchgeführt.

Im allgemeinen setzt man 1 Mol der Verbindung (IV) mit 5 bis 20 Mol Säure, vorzugsweise mit 8 bis 15 Mol Säure um. Die Reaktionsdauer ist von der Temperatur abhängig und liegt zwischen 10 und 20 Stunden.

Eine weitere Herstellungsvariante der Phosphonohydroxyessigsäure (I) beinhaltet die Umsetzung eines Phosphonoesters der allgemeinen Formel (IV), in der $R_1$ und $R_2$ Alkylreste darstellen, mit einem Trialkylsilylhalogenid der Formel (V) (siehe dazu auch individuelles Reaktionsschema B).

Die dafür als Ausgangsstoffe verwendeten Trialkylsilylhalogenide (V) sind ebenfalls bekannt.

In der Formel (V) stehen vorzugsweise $R^3$ für Alkyl mit 1 bis 4 Kohlenstoffatomen, wobei die Alkylgruppen gleich oder verschieden sein können, insbesondere für Methyl, Hal für Chlor, Brom oder Jod, insbesondere für Jod, wenn $R^3$ für Ethyl steht.

Dabei ist es nicht erforderlich, beispielsweise die Trialkylsilyliodide als solche einzusetzen. Es genügt, diese in Gegenwart der Phosphonoester der allgemeinen Formel (IV) aus den entsprechenden Trialkylsilylchloriden und Natriumiodid herzustellen.

Als Verdünnungsmittel kommen für die Umsetzung von Verbindungen der Formel (IV) mit solchen der Formel (V) inerte organische Lösungsmittel in Frage. Geeignete Lösungsmittel sind Kohlenwasserstoffe wie Halogenkohlenwasserstoffe, beispielsweise Tetrachlormethan, oder aromatische Kohlenwasserstoffe, beispielsweise Toluol oder Alkancarbonsäurenitrile mit 2 bis 4 Kohlenstoffatomen, beispielsweise Aceto- oder Propionitril, vorzugsweise Acetonitril.

Die Umsetzung von (IV) mit (V) wird in einem Temperaturbereich zwischen +10° C und +80° C, vorzugsweise zwischen +20° C und +70° C durchgeführt.

Im allgemeinen setzt man 1 Mol der Verbindung (IV) mit 3,0 bis 4,0, vorzugsweise mit 3,0 bis 3,2 Mol der Verbindung (V) um. Es kann auch ein größerer Überschuß an (V) eingesetzt werden.

Die Reaktionsdauer für die Umsetzung von (IV) mit (V) ist von der Temperatur und den eingesetzten Ausgangsverbindungen abhängig und liegt zwischen 15 Minuten und 3 Stunden.

Entsprechend dem Reaktionsschema B wird der Silylester der Verbindung der allgemeinen Formel (VI) mit Wasser umgesetzt.

Die Umsetzung des Silylesters von (VI) mit Wasser wird in einem Temperaturbereich zwischen 0 bis 40° C, vorzugsweise zwischen 20 und 30° C durchgeführt.

Im allgemeinen setzt man 1 Mol des Silylesters der Verbindung (VI) mit mindestens 3 Mol Wasser, zweckmäßig mit einem größeren Überschuß von etwa 30 Mol Wasser um.

Die Reaktionsdauer ist von der Temperatur und von der Struktur der Silylgruppe abhängig und liegt im allgemeinen zwischen 1 Minute und 10 Minuten.

Die erhaltene Verbindung der allgemeinen Formel (VI) wird im allgemeinen in Form ihrer Alkalisalze, beispielsweise des Lithium- oder Natriumsalzes, etwa durch Versetzen mit einer ausreichenden Menge von wäßrigem Lithiumhydroxyd oder Natronlauge und Eindampfen isoliert.

Eine weitere Herstellungsvariante der Phosphonohydroxyessigsäure (I) beinhaltet die Umsetzung einer Verbindung der allgemeinen Formel (IV), in der $R_1$ und $R_2$ Alkylreste darstellen, mit dem Silylierungsreagenz der Formel (VII) oder (VIII) zu der Verbindung (IX), in der $R^4$ vorzugsweise für Alkyl mit 1–4 Kohlenstoffatomen, besonders für Methyl und Ethyl, insbesondere für Methyl steht.

Die Silylierungsreagentien der Formeln (VII) und (VIII) sind bekannt (L. F. Fieser, M. Fieser, Reagents for Organic Synthesis, Band 1, Seite 1235, J. Wiley, London, 1967) oder können nach bekannten Verfahren hergestellt werden.

Beispielsweise seien genannt:

N-Trimethylsilylacetamid, N-Methyl-N-trimethylsilylacetamid, N,O-Bistrimethylsilylacetamid.

Als Verdünnungsmittel kommen für die Umsetzung von (IV) mit dem Silylierungsreagenz (VII) oder (VIII) vor allem die Reaktionspartner inerte organische Lösungsmittel in Frage. Geeignete Lösungsmittel sind vor allem Kohlenwasserstoffe wie Halogenkohlenwasserstoff, beispielsweise Tetrachlormethan oder Chloroform, oder aromatische Kohlenwasserstoffe, beispielsweise Toluol, cyclische Ether, beispielsweise Tetrahydrofuran oder Dioxan, oder Alkancarbonsäurenitrile mit 1 bis 4 Kohlenstoffatomen, beispielsweise Acetonitril oder Propionitril, vorzugsweise Acetonitril.

Die Umsetzung von (IV) mit dem Silylierungsreagenz (VII) oder (VIII) wird in einem Temperaturbereich zwischen +20 und +80° C, vorzugsweise zwischen +30 und +70° C durchgeführt.

Im allgemeinen setzt man 1 Mol der Verbindung (IV) mit 1 bis 3 Mol des Silylierungsreagenz (VII) oder (VIII); vorzugsweise mit 1 bis 2 Mol um. Ein größerer Überschuß schadet nicht.

Die Reaktionsdauer ist von der Temperatur abhängig und liegt zwischen 15 Minuten und 5 Stunden.

Es ist nicht notwendig, die Verbindung (IX) zu isolieren, etwa durch Abdampfen des Verdünnungsmittels, sondern sie kann im geeigneten Verdünnungsmittel direkt weiter umgesetzt werden.

Entsprechend dem Reaktionsschema (C) wird anschließend die erhaltene Verbindung der allgemeinen Formel (IX) mit einem Trialkylsilylhalogenid entsprechend der zweiten Stufe im Reaktionsschema B umgesetzt und der erhaltene silylierte Phosphonoessigsäureester entsprechend der zweiten Stufe im Reaktionsschema B mit Wasser umgesetzt und in Form seiner Salze, z. B. in Form seiner Alkalisalze, z. B. durch Versetzen mit einer ausreichenden Menge wäßriger Natronlauge und Eindampfen isoliert.

Entsprechend den Reaktionsschemata B und C wird in der letzten Stufe der Verfahren die Verbindung der allgemeinen Formel (VI) verseift. Die Verseifung von (VI) kann sauer oder alkalisch erfolgen.

Bei der sauren Verseifung von (VI) verwendet man wäßrige anorganische Säuren, beispielsweise Schwefelsäure oder Salzsäure, vorzugsweise Salzsäure. Bei der alkalischen Verseifung verwendet man als Basen wäßrige Natronlauge oder Kalilauge, vorzugsweise Natronlauge.

Verseift man die Verbindung (VI) alkalisch, so wird das erfindungsgemäße Verfahren in einem Temperaturbereich von 0 bis 100° C, vorzugsweise von 20 bis 40° C durchgeführt. Im sauren Milieu erfolgt die Verseifung bei 20 bis 120° C, vorzugsweise zwischen 90 und 120° C.

Im allgemeinen setzt man 1 Mol der Verbindung (VI) mit 2–7 Mol Säure, vorzugsweise mit 4–6 Mol oder mit 3–5 Mol Lauge, vorzugsweise mit 3–3,5 Mol um.

Die Reaktionsdauer ist von der Temperatur abhängig und liegt zwischen 5 und 24 Stunden.

Alternativ wird die Verbindung der Formel (IV), in welcher $R^1$ und $R^2$ für Aralkyl, insbesondere für Benzyl stehen, in Gegenwart eines Edelmetalls hydrogenolytisch gespalten.

Als Edelmetall eignet sich besonders fein verteiltes Palladium.

Als Verdünnungsmittel kommen vor allem für die Reaktionspartner inerte organische Lösungsmittel in Frage. Geeignete Lösungsmittel sind vor allem aliphatische Ester mit 3 bis 8 Kohlenstoffatomen, wie Essigsäureethylester.

Diese Variante des erfindungsgemäßen Verfahrens wird in einem Temperaturbereich von +20° C bis +150° C, vorzugsweise zwischen +30° C bis +100° C durchgeführt.

Die Reaktion kann bei Normaldruck oder auch bei erhöhtem Druck durchgeführt werden. Im letzteren Fall arbeitet man im allgemeinen bei Drucken zwischen 2 und 100 bar, vorzugsweise zwischen etwa 5 und 50 bar.

Die erhaltene Phosphono-hydroxy-essigsäure der Formel (I) wird beispielsweise in Form ihrer Salze, wie ihrer Alkalisalze, etwa durch Versetzen mit einer ausreichenden Menge wäßriger Natronlauge und Eindampfen isoliert.

Die Phosphono-hydroxy-essigsäure wirkt gegen Viren, insbesondere gegen Herpes-Viren.

Der Wirkstoff kann also solcher oder in Form seiner physiologisch unbedenklichen Salze, beispielsweise von Salzen mit organischen Aminen wie Triethylamin, Cyclohexylamin oder Triethanolamin, oder von Salzen mit anorgansichen Kationen, beispielsweise Lithium, Natrium, Kalium, Magnesium, Calcium, Zink oder Ammonium eingesetzt werden.

Der Wirkstoff kann je nach Verwendungsart beispielsweise lokal, parenteral oder oral verabreicht werden.

Als Zubereitungsform kommen die üblichen galenischen Applikationsformen in Frage, beispielsweise Cremes, Tabletten, Emulsionen, Infusions- und Injektionslösungen.

Besonders eignen sich 0,1- bis 10%ige Formulierungen, vor allem wäßrige Lösungen, z. B. gepufferte Lösungen mit einem pH von 6 bis 8.

Als Dosierungen kommen 10 mg bis 1000 mg/kg Körpergewicht in Frage.

## Herstellungsbeispiele

### Beispiel 1

### Dimethoxyphosphinyl-hydroxy-essigsäurebutylester

Zu 22 g Dimethylphosphit und 26 g Glyoxylbutylester läßt man rasch 1,5 ml einer gesättigten methanolischen Natriumhydroxidlösung einfließen. Die Reaktion verläuft stark exotherm. Man rührt bei 25° einige Stunden nach und erhält durch destillative Aufarbeitung 33,6 g Dimethoxyphosphinylhydroxy-essigsäurebutylester (Ausbeute 70%).

$Kp_{0,05}$ 75–82°C (Dünnschichtverdampfer).
$^1H-NMR$ ($CDCl_3$): $\delta = 0,93$ (3H, t, J = 5 Hz, $CH_3$), 3,86 (6H, d, J = 11 Hz, $CH_3O$), 4,23 (2H, t, J = 6 Hz, $OCH_2$) und 4,63 ppm (1H, d, J = 18 Hz, P—CH).

## Beispiel 2

### Phosphono-hydroxy-essigsäure

27 g Dimethoxyphosphinyl-hydroxy-essigsäurebutylester werden 15 Stunden in 200 ml 5 n-Salzsäure unter Rückfluß gekocht. Man destilliert das Wasser ab, befreit das Produkt durch azeotrope Destillation mit Toluol von restlichem Wasser und erhält 15,0 g Phosphono-hydroxy-essigsäure (Ausbeute: 85%).

$^1H-NMR$ ($D_2O$): $\delta = 4,56$ ppm (1H, d, J = 18 Hz, P—CH).

## Beispiel 3

### Dinatriumsalz des Phosphono-hydroxy-essigsäurebutylesters

15 g Natriumiodid und 12,0 g Dimethoxyphosphinyl-hydroxy-essigsäurebutylester legt man in 100 ml Acetonitril vor und läßt bei 25°C unter Kühlung 16,2 g Trimethylchlorsilan einfließen und rührt anschließend 2 Stunden bei 25°C und 15 Minuten bei 40°C. Das Natriumchlorid wird durch Absaugen oder Zentrifugieren entfernt, die Lösung im Vakuum eingedampft. Der so erhaltene Rückstand wird in 30 ml Wasser aufgenommen, mit verdünnter Natronlauge neutralisiert und anschließend im Vakuum eingedampft. Man erhält nach dem Digerieren in Aceton 12,2 g Phosphono-hydroxy-essigsäurebutylester-Dinatriumsalz.

Ausbeute: 95%.
$^1H-NMR$ ($D_2O$): $\delta = 0,93$ (3H, t, J = 5 Hz, $CH_2$), 4,23 (2H, t, J = 6 Hz, $OCH_2$) und 4,63 ppm (1H, d, J = 17 Hz).

## Beispiel 4

### Trinatriumsalz der Phosphono-hydroxy-essigsäure

17 g Phosphono-hydroxy-essigsäurebutylester-Dinatriumsalz löst man in 60 ml Wasser und gibt 2,6 g Natriumhydroxid zu. Man rührt 24 Stunden bei 20°. Man dampft ein und erhält 14,6 g Phosphono-hydroxy-essigsäure-Trinatriumsalz.

Ausbeute: 98%.
$^1H-NMR$ ($D_2O$): $\delta = 4,07$ ppm (1H, d, J = 18 Hz, P—CH).

## Beispiel 5

### Dinatriumsalz des Phosphono-hydroxy-essigsäurebutylesters

Zu einer Lösung von 12 g Dimethoxyphosphinyl-hydroxy-essigsäurebutylester in 50 ml Acetonitril läßt man bei 25° 7,2 g N-Trimethylsilyl-N-methylacetamid in 20 ml Acetonitril einfließen. Man erwärmt dann 4 Stunden auf 60°C. Bei 25°C versetzt man mit 15 g Natriumiodid und läßt 10,8 g Trimethylchlorsilan unter Kühlung bei 25°C einfließen. Ansonsten verfährt man wie unter Beispiel 3 beschrieben. Man erhält 9,6 g Phosphono-hydroxy-essigsäurebutylester-Dinatriumsalz.

Ausbeute: 75%.

## Beispiel 6

Die Umsetzung von Phosphono-hydroxy-essigsäurebutylester-Dinatriumsalz mit einer wäßrigen NaOH-Lösung erfolgt in Analogie zu Beispiel 4. Das gemäß Beispiel 4 bzw. Beispiel 8 erhaltene Trinatriumsalz der Phosphono-hydroxy-essigsäure wird gegebenenfalls mit der entsprechenden

Menge Säure in die freie Phosphonohydroxyessigsäure übergeführt.

Beispiel 7

Dibenzyloxyphosphinyl-hydroxy-essigsäurebenzylester

Zu 45,5 g Dibenzylphosphit und 28,5 g Glyoxylsäurebenzylester läßt man rasch 1,5 ml einer gesättigten methanolischen Natriumhydroxidlösung einfließen. Die Reaktion verläuft exotherm. Man rührt einige Stunden bei 20° C nach. Es wird an Kieselgel mit 5% Methanol enthaltendem Methylenchlorid chromatographiert. Die Fraktion mit dem $R_f=0,58$ dampft man ein, versetzt den Rückstand (55,2 g) mit 20 ml Diethylether und isoliert 45,0 g Dibenzyloxyphosphinylhydroxy-essigsäurebenzylester. Fp. 67—68° C.

Ausbeute: 61%.

Beispiel 8

Trinatriumsalz der Phosphono-hydroxy-essigsäure

4,3 g Dibenzyloxyphosphinyl-hydroxy-essigsäurebenzylester legt man in 150 ml Essigsäureethylester vor, gibt 1 g Palladium auf Kohle (5%) zu und hydriert bei Normaldruck und 20° C. Nach Aufnahme der theoretischen Menge Wasserstoff trennt man den Katalysator ab, dampft im Vakuum ein, nimmt den Rückstand in 15 ml Wasser auf, stellt mit 2n Natronlauge auf pH 8 ein und dampft erneut im Vakuum bis zur Trockne ein. Man erhält 2,2 g Trinatriumsalz der Phosphono-hydroxy-essig säure (Ausbeute: quantitativ).

IR (KBr): $\nu = 970, 1080, 1405$ und $1590\ cm^{-1}$.

Beispiel 9

Dilithiumsalz des Phosphono-hydroxy-essigsäurebutylesters

Man verfährt wie in Beispiel 3 beschrieben. Statt mit verdünnter Natronlauge neutralisiert man mit 2 n-Lithiumhydroxydlösung. Das Dilithiumsalz des Phosphono-hydroxy-essigsäurebutylesters fällt aus (Ausbeute: 85%).

IR (KBr): $\nu = 1710\ cm^{-1}$.

Beispiel 10

Trilithiumsalz der Phosphono-hydroxy-essigsäure

22 g Dilithiumsalz des Phosphono-hydroxy-essigsäurebutylesters versetzt man mit 20 ml Wasser und gibt 2,4 g Lithiumlauge zu. Man rührt 48 Stunden bei 20°. Man dampft ein und erhält nach dem Waschen mit Ethanol 14 g Trilithiumsalz der Phosphono-hydroxy-essigsäure (Ausbeute: 80%).

IR (KBr): $\nu = 985, 1110, 1425$ und $1600\ cm^{-1}$.

**Patentansprüche**

1. Antivirale Mittel, gekennzeichnet durch einen Gehalt an Phosphono-hydroxy-essigsäure der Formel

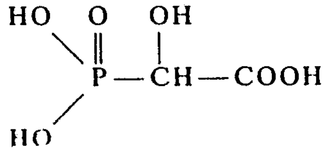

(I)

und/oder ihrer physiologisch verträglichen Salze.

2. Antivirale Mittel nach Anspruch 1, gekennzeichnet durch einen Gehalt an physiologisch verträglichen Salzen der Phosphono-hydroxy-essigsäure der Formel I mit organischen Aminen.

3. Antivirale Mittel nach Anspruch 1, gekennzeichnet durch einen Gehalt an physiologisch verträglichen Salzen der Phosphono-hydroxy-essigsäure der Formel I mit anorganischen Kationen.

4. Verfahren zur Herstellung von Phosphono-hydroxy-essigsäure der Formel

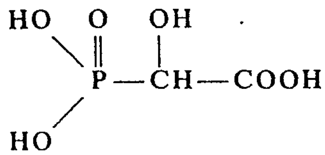

(I)

dadurch gekennzeichnet, daß man Verbindungen der Formel

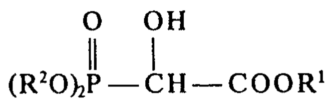

(IV)

in welcher
$R^1$ und $R^2$ gleich oder verschieden sein können und für Alkyl stehen oder $R^1$ und $R^2$ gleich sind und für Aralkyl stehen,

a) im Falle, daß $R^1$ und $R^2$ für Alkyl stehen, entweder in Gegenwart einer Säure mit Wasser umsetzt oder nach Umsetzung mit Trialkylsilylhalogeniden bzw. mit Mono- oder Bis-Trialkylsilyl-niedrigalkylcarbonsäureamiden und darauf mit Trialkylsilylhalogeniden im wasserfreien Medium, anschließend mit Wasser in Gegenwart von Säuren bzw. von Basen umsetzt, oder
b) im Falle, daß $R^1$ und $R^2$ für Aralkyl stehen, mit Wasserstoff in Gegenwart eines Edelmetallkatalysators umsetzt,

und gegebenenfalls die physiologisch verträglichen Salze herstellt.

5. Verfahren zur Herstellung von Arzneimitteln gemäß Anspruch 1—3, dadurch gekennzeichnet, daß man Phosphono-hydroxy-essigsäure oder ihre physiologisch verträglichen Salze gemäß Anspruch 1 mit inerten, pharmazeutisch verträglichen Hilfs- und/oder Zusatzstoffen vermischt.

**Claims**

1. Antiviral agents, characterised in that they contain phosphonohydroxyacetic acid of the formula

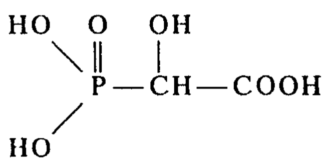

(I)

and/or physiologically acceptable salts thereof.

2. Antiviral agents according to Claim 1, characterised in that they contain physiologically acceptable salts of the phosphonohydroxyacetic acid of the formula I with organic amines.

3. Antiviral agents according to Claim 1, characterised in that they contain physiologically acceptable salts of the phosphonohydroxyacetic acid of the formula I with inorganic cations.

4. Process for the preparation of phosphonohydroxyacetic acid of the formula

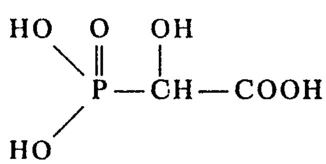

(I)

characterised in that compound of the formula

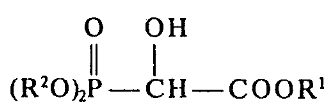

(IV)

in which
$R^1$ and $R^2$ can be identical or different and represent alkyl or $R^1$ and $R^2$ are identical and represent aralkyl,

a) in the case where $R^1$ and $R^2$ represent alkyl, are either reacted with water in the presence of an acid or after reaction with trialkylsilyl halides or with mono- or bis-trialkylsilyl-lower alkyl-carboxylic acid amides and then with trialkylsilyl halides in an anhydrous medium, are then reacted with water in the presence of acids or bases, or
b) in the case where $R^1$ and $R^2$ represent aralkyl, are reacted with hydrogen in the presence of a noble metal catalyst,

and the physiologically acceptable salts are optionally prepared.

5. Process for the preparation of medicaments according to Claim 1—3, characterised in that phosphonohydroxyacetic acid, or physiologically acceptable salts thereof, according to Claim 1 is mixed with inert, pharmaceutically acceptable auxiliaries and/or additives.

## Revendications

1. Agents antiviraux, caractérisés en ce qu'ils contiennent l'acide phosphono-hydroxy-acétique de formule:

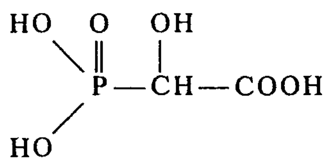

(I)

et/ou ses sels physiologiquement compatibles.

2. Agents antiviraux suivant la revendication 1, caractérisés en ce qu'ils contiennent des sels physiologiquement compatibles de l'acide phosphono-hydroxy-acétique de formule I avec des amines organiques.

3. Agents antiviraux suivant la revendication 1, caractérisés en ce qu'ils contiennent des sels physiologiquement compatibles de l'acide phosphono-hydroxy-acétique de formule I avec des cations inorganiques.

4. Procédé de préparation de l'acide phosphono-hydroxy-acétique de formule:

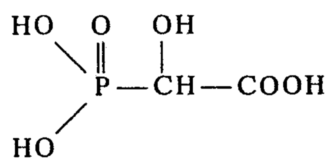

(I)

caractérisé en ce qu'on fait réagir des composés de formule:

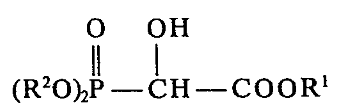

(IV)

dans laquelle $R^1$ et $R^2$ peuvent être identiques ou différents et représentent chacun un groupe alkyle ou $R^1$ et $R^2$ sont identiques et représentent un groupe aralkyle,

a) avec de l'eau en présence d'un acide si $R^1$ et $R^2$ représentent chacun un groupe alkyle ou, après la réaction avec des halogénures de trialkyl-silyle ou avec des amides d'acides mono- ou bis-trialkylsilyl-alkyl inférieur-carboxyliques, puis avec des halogénures de trialkyl-silyle en milieu anhydre et ensuite avec de l'eau en présence d'acides ou de bases, ou
b) avec de l'hydrogène en présence d'un catalyseur d'un métal noble si $R^1$ et $R^2$ représentent chacun un groupe aralkyle,

et l'on prépare éventuellement les sels physiologiquement compatibles.

5. Procédé de préparation de médicaments suivant les revendications 1 à 3, caractérisé en ce qu'on mélange l'acide phosphono-hydroxy-acétique ou ses sels physiologiquement compatibles suivant la revendication 1 avec des agents auxiliaires et/ou des additifs inertes et pharmaceutiquement compatibles.